# FASCICULE DE BREVET EUROPEEN

(11) **EP 0 974 316 B1**
(45) Date de publication et mention de la délivrance du brevet: **05.02.2003**
(21) Numéro de dépôt: 99401869.5
(22) Date de dépôt: 23.07.1999
(51) Int. Cl.: A61F 2/34

(54) **Cotyle pour prothèse de hanche**
Gelenkpfanne für Hüftprothese
Acetabular cup for hip prosthesis

(30) Priorité: 24.07.1998 FR 9809496
(43) Date de publication de la demande: 26.01.2000
(73) Titulaire: Fessy, Michel Henry, 69390 Millery (FR); Anania, Gaetano, 89100 Reggio Calabria (IT); Chatelet, Jean-Christophe, 01150 Chazey Sur Ain (FR); Denjean, Stéphane, 71960 Sommere Par La Roche Vineuse (FR); Gaillard, Thierry, 69003 Lyon (FR)
(72) Inventeur: Fessy, Michel Henry, 69390 Millery (FR); Anania, Gaetano, 89100 Reggio Calabria (IT); Chatelet, Jean-Christophe, 01150 Chazey Sur Ain (FR); Denjean, Stéphane, 71960 Sommere Par La Roche Vineuse (FR); Gaillard, Thierry, 69003 Lyon (FR)
(74) Mandataire: Robert, Jean-Pierre

(56) Documents cités:
- EP-A- 0 499 475
- EP-A- 0 532 439
- FR-A- 2 708 459

## Description

La présente invention concerne un cotyle pour prothèse articulaire de hanche.

Une prothèse de hanche comporte principalement une tige d'ancrage destinée à être introduite dans le fût fémoral pour y être scellée soit de façon naturelle par ostéointégration soit artificiellement au moyen d'un ciment, et une tête articulaire sphérique qui est destinée à se substituer à la tête fémorale naturelle pour être reçue dans la cavité cotyloïdienne de l'os iliaque correspondant du patient. La tête fémorale naturelle ayant été préalablement réséquée, la tige d'ancrage possède une extrémité ou col qui s'étend en saillie du bord réséqué du fémur pour être raccordée à la tête articulaire sphérique.

La tête articulaire peut être réalisée de deux manières différentes selon le type de pathologie rencontré. Lorsque la cavité cotyloïdienne n'est pas trop endommagée, on peut utiliser une tête articulaire simple en une pièce solidaire du col de la tige d'ancrage, qui est mobile dans la cavité cotyloïdienne et qui ne fait donc que se substituer à la tête fémorale naturelle. Lorsque la cavité cotyloïdienne est trop endommagée pour continuer à remplir sa fonction d'appui glissant avec la tête fémorale, il est nécessaire d'utiliser une tête articulaire réalisant intrinsèquement une liaison rotule. Ce second type de tête articulaire prothétique comporte d'une part un cotyle artificiel hémisphérique qui est fixé à demeure dans la cavité cotyloïdienne naturelle du patient, et d'autre part une pièce sphérique (ou rotule) solidaire du col et reçue dans un logement hémisphérique correspondant du cotyle artificiel pour réaliser une liaison rotule par glissement de la pièce sphérique dans le logement du cotyle.

Un cotyle artificiel utilisé dans ce genre de prothèse comporte classiquement une cupule métallique présentant une forme hémisphérique évidée, avec un pôle et un bord équatorial, et un noyau (ou insert) de glissement, généralement en polyéthylène, reçu dans la cupule et dans lequel est ménagé le logement hémisphérique pour la liaison articulée du cotyle avec la pièce sphérique portée par le col de la tige fémorale. La cupule présente en outre des griffes intérieures adjacentes au bord équatorial et faisant saillie à l'intérieur de la cupule pour mordre directement le noyau de glissement.

Dans les cotyles actuellement disponibles sur le marché, les griffes sont ménagées sur toute la périphérie de la cupule. Il en résulte que le noyau de glissement est marqué en correspondance extérieurement sur toute sa périphérie dès qu'il est inséré dans la cupule. Il est donc difficile de modifier la position angulaire relative du noyau de glissement dans la cupule pour l'ajuster car l'empreinte des griffes de la position modifiée risque d'interférer avec celle de la position initiale.

Une cotyle comprenant les caractéristiques du préambule de la revendication 1 est connue du document FR-A-2 708 459.

Un but de l'invention est de proposer un cotyle dont les moyens de fixation du noyau de glissement dans la cupule permettent une modification aisée, précise et sûre de la position angulaire du noyau de glissement dans la cupule.

En vue de la réalisation de ce but on propose selon l'invention un cotyle comprenant une cupule de forme hémisphérique avec un pôle et un bord équatorial, et présentant intérieurement un logement hémisphérique, et un noyau définitif de glissement au moins partiellement reçu dans le logement de la cupule présentant un logement interne hémisphérique pour recevoir en appui glissant une rotule de tige fémorale, la cupule présentant des griffes intérieures d'ancrage adjacentes au bord équatorial et faisant saillie à l'intérieur de la cupule pour mordre directement le noyau de glissement, les griffes étant réparties en plusieurs groupes espacés les uns des autres.

Ainsi, lorsque le noyau de glissement est inséré pour la première fois dans la cupule, les griffes de cette dernière ne marquent le noyau de glissement que sur certaines parties de sa périphérie, laissant vierge de toute empreinte le reste de sa périphérie. Si la position angulaire du noyau de glissement dans la cupule doit être modifiée pour un ajustement, il suffit de retirer le noyau de glissement et de le réinsérer dans la position souhaitée. Les griffes, ou au moins certaines d'entre elles, viennent alors mordre des parties de la périphérie du noyau de glissement qui sont restées vierges, sans interférer avec les anciennes empreintes laissées par les griffes. On obtient de ce fait une fixation sûre et précise du noyau de glissement dans la cupule, même après modification de la position du noyau de glissement.

Selon une caractéristique avantageuse de l'invention, les groupes de griffes sont régulièrement espacés les uns des autres. On obtient ainsi un ancrage homogène du noyau de glissement dans la cupule.

Dans un mode de réalisation avantageux, la cupule comporte quatre groupes de griffes et chaque groupe comporte quatre griffes.

Lors de l'implantation de la prothèse, la cupule du cotyle est d'abord mise en place dans la cavité cotyloïdienne sans grande précision quant à son orientation autour de son axe. En revanche, lorsque le noyau de glissement est installé dans la cupule, son orientation autour de l'axe de la cupule n'est pas indifférente, car la face avant du rebord d'appui est inclinée par rapport au bord équatorial de la cupule. Cette orientation doit être déterminée avec soin en fonction de l'anatomie du patient pour préserver au maximum sa mobilité et minimiser les risques de douleurs. Actuellement cette opération de réglage s'effectue par tâtonnement par plusieurs mises en place et retraits successifs du noyau définitif dans la cupule. Or, si la mise en place du noyau ne pose généralement aucune difficulté particulière, son retrait est souvent laborieux et peut même parfois, selon le mode d'ancrage du noyau dans la cupule (en particulier dans le cas d'une cupule à griffes mordant le noyau), aboutir à une dégradation de la résistance de l'ancrage par usure ou même détérioration du noyau.

Un but de l'invention est de concevoir un cotyle incorporant des moyens pour faciliter le réglage de la position angulaire du noyau de glissement dans la cupule autour de l'axe de la cupule, sans endommager le noyau de glissement.

En vue de la réalisation de ce but, on prévoit, selon l'invention, un cotyle pour prothèse articulaire de hanche, comportant :
- une cupule de forme hémisphérique évidée, avec un pôle et un bord équatorial, et présentant intérieurement un logement hémisphérique, et
- un noyau définitif de glissement présentant un logement interne hémisphérique pour recevoir en appui glissant une rotule de tige fémorale et comprenant une partie sensiblement hémisphérique conformée pour être reçue dans le logement de la cupule et un rebord d'appui en débordement de cette cupule,
le cotyle comportant un noyau d'essai qui est temporairement substituable au noyau définitif de glissement et qui, comme ce dernier, présente un logement interne hémisphérique et comporte une partie sensiblement hémisphérique conformée pour être reçue dans le logement de la cupule et un rebord d'appui en débordement de cette cupule, le noyau temporaire d'essai possédant des moyens de sa liaison temporaire à la cupule agencés pour coopérer avec le taraudage central de la cupule et pour permettre un pivotement avec frottement du noyau d'essai dans le logement de la cupule en vue de déterminer la position angulaire adéquate du noyau définitif de glissement.

D'autres caractéristiques et avantages de l'invention apparaîtront à la lecture de la description qui suit d'un mode de réalisation particulier donné à titre d'exemple non limitatif.

Il sera fait référence aux dessins en annexe parmi lesquels :
- la figure 1 est une vue en perspective éclatée d'un cotyle, avec sa cupule et son noyau définitif de glissement, conforme à l'invention ;
- la figure 2 est une vue de face de la cupule seule, de son côté intérieur ;
- la figure 3 est une vue en coupe axiale du cotyle, avec sa cupule et son noyau définitif de glissement ancré dans le logement interne de la cupule, le taraudage central de la cupule étant obturé par le bouchon fileté ;
- la figure 4 est une vue de détail en perspective du bouchon fileté seul ;
- la figure 5 est une vue de la face arrière du bouchon fileté avec son empreinte ;
- la figure 6 est une vue en coupe axiale du cotyle, avec sa cupule, et son noyau temporaire d'essai attelé au taraudage central.

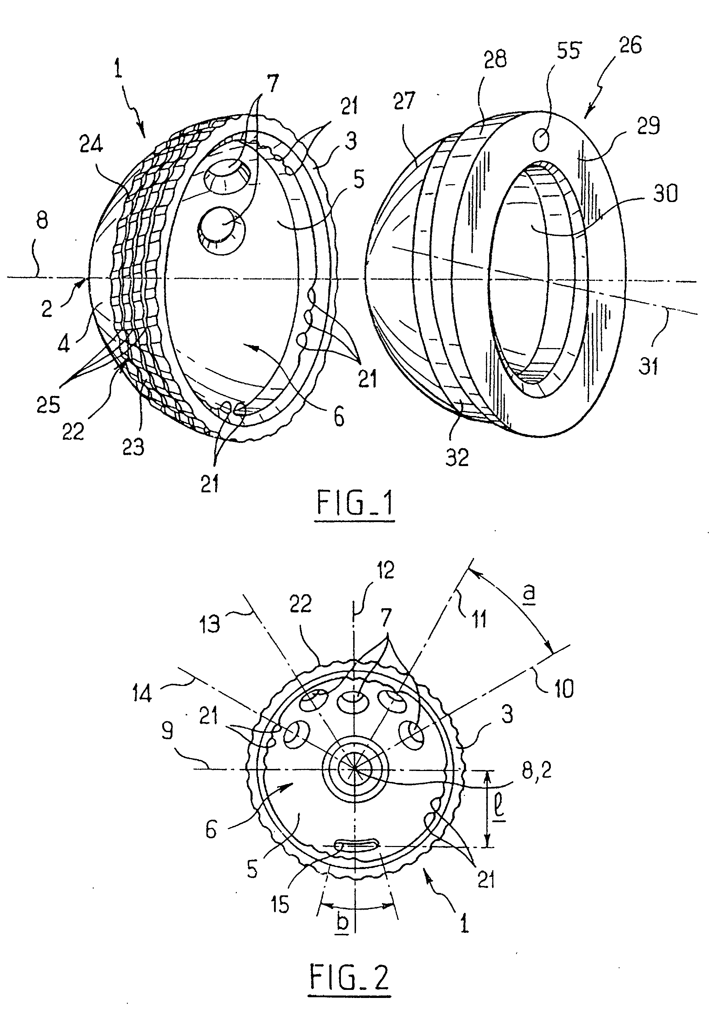

En référence aux figures, et en particulier aux figures 1 à 3, un cotyle pour prothèse articulaire de hanche conforme à l'invention comporte une cupule 1 présentant une forme sensiblement hémisphérique évidée ayant un axe de révolution 8, un pôle 2 et un bord équatorial 3. Cette cupule peut par exemple être réalisée en un matériau métallique tel qu'un alliage de titane et peut être recouvert sur sa face extérieure 4 d'un revêtement d'hydroxyapatite favorisant dans la cavité cotyloïdienne, une ostéointégration au contact de la cupule. De son côté intérieur, la cupule présente une face intérieure 5 qui délimite un logement hémisphérique 6.

La cupule 1 comporte une pluralité de trous 7 servant à sa fixation dans la cavité cotyloïdienne au moyen de vis (non représentés aux figures) qui sont engagées dans l'os spongieux au travers de ces trous.

Comme cela est mieux visible à la figure 2, les trous de fixation 7 sont tous situés sur une moitié de la cupule. Autrement dit, si l'on divise la cupule 1 en deux moitiés séparées par un méridien 9 qui est en l'espèce horizontal, les trous de fixation 7 sont tous situés au-dessus de ce méridien 9, c'est-à-dire dans la moitié supérieure de la cupule 1.

Plus précisément, les trous 7 sont ici au nombre de cinq, et sont de forme conique évasée vers l'intérieur de la cupule. Ils sont régulièrement espacés les uns des autres, de telle sorte que leurs centres soient situés sur des méridiens 10, 11, 12, 13, 14 formant deux à deux un angle a d'environ 30°. Les cinq trous 7 sont ainsi situés dans un secteur d'environ 120°. En outre, les trous 7 sont organisés symétriquement de part et d'autre du méridien 12 qui passe par le centre du troisième trou 7, qui est le trou médian, et sont disposés en quinconce sur deux rangées parallèles au bord équatorial 3, les deux trous extrêmes et le trou médian étant situés sur la rangée la plus éloignée du bord 3 et les deux autres trous situés entre les trous extrêmes et le trou médian étant situés sur la rangée la plus proche du bord 3.

Par ailleurs, la cupule 1 possède une fenêtre de repérage visuel 15 qui est située dans l'autre moitié (inférieure) de la cupule, c'est-à-dire en-dessous du méridien 9. Cette fenêtre permet, lorsque la cupule 1 est implantée dans la cavité cotyloïdienne, de contrôler visuellement la position et l'orientation de la cupule afin, éventuellement, de la corriger.

En l'espèce, la fenêtre 15 est de forme oblongue suivant une direction sensiblement parallèle au bord équatorial 3 de la cupule et s'étend suivant cette direction sur un secteur d'angle b qui est en l'espèce voisin de 30°. De plus, la fenêtre 15 est ici centrée sur le même méridien de symétrie 12 que les trous de fixation 7. Enfin, on a pu déterminer aux essais qu'une visualisation optimum était obtenue pour un positionnement du centre de la fenêtre 15 à une latitude 1 d'environ 57° par rapport au pôle 2 de la cupule.

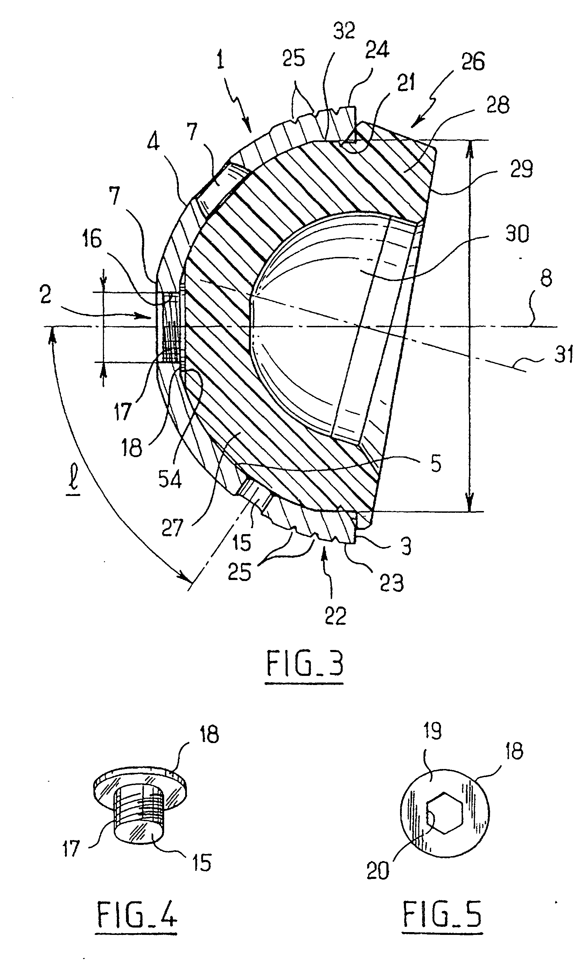

Au voisinage de son pôle 2, la cupule 1 présente un taraudage central débouchant 16, mieux visible à la figure 3, qui est apte à recevoir l'extrémité filetée d'un outil de montage (non représenté aux figures) permettant la manipulation de la cupule 1 en vue de sa mise en place dans la cavité cotyloïdienne. Le taraudage débouchant 16 peut être obturé, après la mise en place de la cupule dans la cavité cotyloïdienne, par un bouchon fileté 17 qui, comme cela est visible aux figures 3 à 5, possède une tête 18 en forme de disque reçue dans un lamage correspondant 54 ménagé en renfoncement de la face intérieure 5 de la cupule 1 autour de l'axe 8. La tête 18 du bouchon 17 présente en outre une face arrière 19 qui, lorsque le bouchon 17 est reçu dans le taraudage 16, est située à l'intérieur de la cupule 1 et en renfoncement de laquelle est ménagée une empreinte 20, en l'espèce hexagonale, pour coopérer avec l'extrémité correspondante d'un tournevis (non représenté).

La cupule 1 est d'autre part pourvue de griffes d'ancrage 21 qui sont adjacentes au bord équatorial 3 et qui font saillies radialement à l'intérieur de la cupule 1. Comme cela est visible aux figures 1 et 2, ces griffes 21 sont réparties par groupes régulièrement espacés les uns des autres. En l'espèce, la cupule comporte quatre groupes de quatre griffes 21 chacun.

La cupule 1 est enfin pourvue sur sa face extérieure 4 d'une bande annulaire cannelée 22 qui est adjacente au bord équatorial 3 et qui sert à l'ancrage de la cupule 1 dans la cavité cotyloïdienne. Les cannelures de cette bande annulaire d'ancrage sont inclinées par rapport au méridien de la cupule. Plus précisément, comme cela est visible seulement à la figure 1, la bande annulaire d'ancrage 22 se divise en deux portions d'égale longueur sur chacune desquelles les cannelures sont parallèles entre elles et sont inclinées en sens inverse des cannelures de l'autre portion. On distingue ainsi une série de cannelures 23 inclinées d'un certain angle par rapport au méridien de la cupule et formant une première portion, et une série de cannelures 24 inclinées par rapport au méridien de la cupule d'un angle inverse de celui des cannelures 23 et formant la seconde portion de la bande 22.

De plus, la bande annulaire d'ancrage 22 présente des rainures annulaires 25 qui s'étendent parallèlement au bord équatorial 3 sur toute la circonférence de la cupule 1. Ces rainures annulaires 25 s'opposent à l'extraction de la cupule selon une direction parallèle à son axe de révolution 8 et renforcent encore l'obstruction au pivotement de la cupule 1 dans la cavité cotyloïdienne autour d'un axe perpendiculaire à son axe de révolution 8.

En référence plus particulièrement aux figures 1 et 3, le cotyle comporte également un noyau définitif de glissement 26, qui peut être réalisé en un matériau synthétique tel que du polyéthylène et qui sert d'interface de glissement et d'amortissement entre la cupule et la rotule de tige fémorale de la prothèse (non représentée aux figures).

Ce noyau, parfois également appelé insert, comprend une partie 27 de forme sensiblement hémisphérique, conformée pour être reçue sans jeu dans le logement 6 de la cupule 1 et un rebord d'appui 28 qui, lorsque la partie 27 est reçue dans le logement 6 de la cupule, vient en débordement de cette cupule, en appui contre son bord équatorial 3. Ce rebord 28 présente une face avant 29 qui s'étend dans un plan légèrement incliné par rapport au bord équatorial 3 de la cupule, c'est-à-dire par rapport à un plan perpendiculaire à l'axe 8 de la cupule. Cette face 29 porte une marque de repérage 55 dont la fonction sera mieux expliquée ultérieurement.

Un logement interne 30, de forme sensiblement hémisphérique, est ménagé dans le noyau 26, en renfoncement de sa face avant 29. Ce logement hémisphérique 30 est destiné à recevoir en appui glissant la rotule de tige fémorale (qui est une pièce sphérique non représentée aux figures) et est ménagé suivant un axe de révolution 31 qui est incliné par rapport à l'axe 8 de la cupule 1 et qui, en l'espèce, est sensiblement perpendiculaire à la face avant 29 du noyau 26.

Pour son ancrage dans le logement 6 de la cupule 1, la partie hémisphérique 27 du noyau 26 présente, au voisinage du rebord 28, une portion annulaire d'ancrage 32, sensiblement cylindrique ou légèrement tronconique, que les griffes 21 viennent mordre lorsque la partie 27 est introduite dans la cupule. Le noyau 26 est ainsi non seulement retenu dans la cupule mais est de plus immobilisé en rotation par rapport à celle-ci autour de l'axe 8.

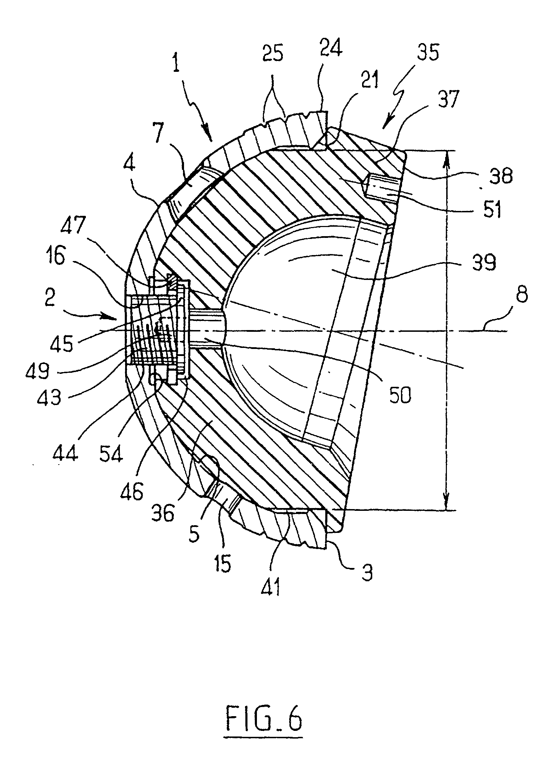

En référence à la figure 6, le cotyle comporte d'autre part un noyau d'essai 35 qui est temporairement substituable au noyau définitif de glissement 26 avant que ce dernier ne soit définitivement mis en place et ancré dans la cupule 1. Ce noyau temporaire d'essai permet de simuler la configuration du cotyle in situ aux fins d'un réglage précis et commode de la position angulaire du noyau dans la cupule autour de son axe 8. Ce noyau temporaire d'essai permet également de vérifier que la tige d'ancrage reçue dans le fémur du patient, une fois sa tête reçue dans le noyau d'essai, a des caractéristiques dimensionnelles, telles que la longueur de son col, adaptées au patient.

Comme le noyau définitif de glissement 26, le noyau temporaire d'essai 35 comporte une partie 36 de forme sensiblement hémisphérique conformée pour être reçue dans le logement 6 de la cupule 1 et un rebord d'appui 37 qui, lorsque la partie 36 est introduite dans la cupule, vient en débordement de celle-ci, en appui contre son bord équatorial 3. Le noyau temporaire d'essai 35 présente de plus une face avant 38 inclinée par rapport au bord équatorial 3 et un logement interne hémisphérique 39 qui sont tous deux agencés de façon identique à la face 29 et au logement 30 du noyau définitif 26.

A la différence du noyau définitif 26, la partie 36 du noyau temporaire d'essai 35 présente au voisinage du rebord 37 une portion annulaire cylindrique 41 dont le diamètre est plus petit que celui de la portion d'ancrage 32 du noyau définitif 26, de façon à ne pas venir en prise avec les griffes d'ancrage 21 de la cupule 1 afin d'éviter un ancrage rigide du noyau temporaire d'essai 35 et surtout de préserver la liberté de pivotement de ce noyau autour de l'axe 8.

Le noyau temporaire d'essai 35 est attelé à la cupule 1 au moyen d'une vis 43. Cette vis possède une tête plate 45 qui forme un épaulement reçu dans une cavité 46 ménagée dans l'épaisseur du noyau d'essai 35 et retenu axialement dans cette cavité, tout en pouvant pivoter librement autour de son axe (qui est confondu avec l'axe 8 de la cupule 1), par un circlips 47 engagé dans une rainure périphérique correspondante de la cavité 46. La vis 43 possède de plus une partie filetée 44 qui fait saillie à l'extérieur de la partie 36 du noyau d'essai 35 pour être reçue dans le taraudage central 16 de la cupule 1. Le serrage de la vis 46 est ajusté de manière à permettre un pivotement avec frottement du noyau temporaire d'essai 35 dans le logement 6 de la cupule 1. A cet effet, bien que cela ne soit pas prévu dans l'exemple illustré à la figure 3, on pourra prévoir que la vis 43 ou le taraudage central 16 de la cupule 1 soit pourvu d'une butée telle qu'un épaulement permettant de limiter l'intensité du serrage de la vis 43 à la force appropriée.

En outre, pour permettre son vissage au moyen d'un tournevis adapté, la vis 43 présente, en renfoncement de la face arrière de sa tête 45, une empreinte 49. Cette empreinte 49 est accessible, depuis le logement interne 39 du noyau temporaire d'essai 35, par un passage 50 qui débouche dans la cavité 46 et qui est centré sur l'axe 8.

Par ailleurs, la face avant 38 du rebord 37 du noyau temporaire d'essai 35 présente un repère visuel 51, ici constitué par un perçage, qui permet de repérer de façon exacte et commode la position angulaire du noyau temporaire d'essai 35 par rapport à la cupule 1 autour de l'axe 8. Ce repère 51 est situé au même emplacement que la marque 55 du noyau définitif de glissement 26.

L'implantation du cotyle qui vient d'être décrite dans la cavité cotyloïdienne d'un patient se déroule selon le mode opératoire suivant. La cupule 1 est introduite seule, au moyen d'un outil (ancillaire) dont l'extrémité filetée est en prise dans le taraudage central 16, dans la cavité cotyloïdienne. Des vis de fixation sont introduites au travers des trous 7 pour être vissées dans l'os spongieux de manière à ancrer de façon relativement rigide la cupule 1 dans la cavité cotyloïdienne. Lorsque le positionnement et l'ancrage de la cupule 1 sont satisfaisants, l'outil de manipulation est retiré en dévissant son extrémité filetée du taraudage 16 et ce taraudage 16 est obturé par le bouchon fileté 17. On notera que le positionnement de la cupule 1 dans la cavité cotyloïdienne est facilité par la présence en partie inférieure de cette cupule de la fenêtre de visualisation 15 dont la position sur la cupule correspond à l'emplacement des repères anatomiques qui peuvent être utilisés dans ce genre d'opération.

On notera que, grâce à leur inclinaison, les cannelures 23, 24 de la bande annulaire d'ancrage 22 constituent un obstacle au pivotement de la cupule non seulement autour de son axe de révolution 8 mais également autour d'un axe perpendiculaire à celui-ci, ce qui assure, après ostéointégration, une immobilisation complète de la cupule 1 dans la cavité cotyloïdienne. Cette immobilisation est renforcée par la double inclinaison des cannelures, les unes en sens opposé des autres.

La cupule étant ainsi mise en place, le positionnement et la mise en place du noyau dans la cupule peuvent alors commencer.

Dans les cas (relativement fréquents) où la position angulaire du noyau dans la cupule autour de l'axe de cette cupule doit être réalisée avec précision, il est préférable d'utiliser le noyau temporaire d'essai 35 avant de mettre en place le noyau définitif 26. Le cas échéant, le noyau temporaire d'essai 35 est donc préalablement installé dans la cupule 1. Son attelage à cette cupule est réalisé au moyen de la vis 43 dont la partie filetée 44 est engagée avec le taraudage central 16. La vis 43 étant serrée de manière à obtenir une liaison pivotante avec frottement du noyau temporaire d'essai 35 avec la cupule 1, le réglage de la position angulaire de ce noyau autour de l'axe 8 peut être effectué avec commodité et précision. Le repère visuel 51 présent sur la face avant 38 du noyau temporaire d'essai 35 facilite le repérage visuel de la position souhaitée. Eventuellement, le perçage 51 (qui sert de repère visuel) peut également servir d'élément d'indexation en coopérant avec un outil (ancillaire) de manipulation associé. Lorsqu'une position satisfaisante est obtenue en fonction de l'anatomie du patient, cette position est repérée ou marquée à l'aide du repère visuel 51 et le noyau temporaire d'essai 35 peut être retiré en dévissant la vis 43 du taraudage central 16.

La position angulaire du noyau étant ainsi prédéterminée, le noyau définitif 26 peut être mis en place sur la cupule 1. A cet effet, la partie 27 du noyau 26 est introduite dans le logement 6 de la cupule 1 de façon à ce que sa partie d'ancrage 32 vienne affleurer les griffes 21 de la cupule 1. La position angulaire du noyau 26 autour de l'axe 8 est ensuite ajustée. Cet ajustement est effectué en fonction de la position prédéterminée à l'aide du noyau temporaire d'essai 35 comme décrit ci-dessus, une marque 55 de la face avant 29 du noyau 26 devant venir dans la même position que le repère visuel 51 du noyau temporaire d'essai 35.

Lorsque la bonne position angulaire est obtenue, le noyau définitif 26 est emboîté à force dans le logement 6 de la cupule 1, les griffes 21 venant mordre la portion d'ancrage 32 de la partie 27 de ce noyau.

On notera ici que le fait que les griffes 21 soient réparties par groupes espacés les uns des autres permet d'éviter que ces griffes ne marquent la portion d'ancrage 31 du noyau sur toute sa périphérie. Ainsi, lorsque la position angulaire du noyau 26 par rapport à la cupule 1 doit être modifiée pour un ajustement, il suffit de retirer le noyau et de le réinsérer dans la position souhaitée, les griffes 21, ou au moins certaines d'entre elles venant alors mordre des parties de la portion d'ancrage 31 du noyau qui sont restées vierges, sans interférer avec les anciennes empreintes laissées par les griffes dans la position précédemment jugée insatisfaisante. L'ancrage du noyau 26 dans la cupule 1 est de ce fait plus sûr et plus précis, même après une éventuelle modification de la position angulaire du noyau.

D'autre part, le fait que les trous de fixation 7 de la cupule soient seulement situés sur la partie supérieure de la cupule permet d'améliorer la répartition des efforts d'appui de la partie hémisphérique 27 du noyau 26 contre la face intérieure 5 5 de la cupule 1, ce qui minimise le risque de fluage de la matière constituant le noyau (telle que du polyéthylène) au travers des trous de fixation inutilisés, c'est-à-dire au travers desquels aucune vis de fixation n'est introduite. De même, l'obturation du taraudage central 16 par le bouchon fileté 17 permet d'éviter tout risque de fluage de la matière constituant le noyau 26 au travers du taraudage 16, la tête plate 18 du bouchon 17 offrant de plus une surface d'appui supplémentaire pour la partie 27 du noyau 26, en continuité avec la surface intérieure 5 de la cupule 1.

## Revendications

1. Cotyle pour prothèse articulaire de hanche, comportant :
- une cupule (1) de forme hémisphérique évidée, avec un pôle (2) et un bord équatorial (3), et présentant intérieurement un logement hémisphérique (6), et
- un noyau définitif de glissement (26) au moins partiellement reçu dans le logement (6) de la cupule (1) et présentant un logement interne hémisphérique (30) pour recevoir en appui glissant une rotule de tige fémorale,
la cupule (1) présentant des griffes intérieures d'ancrage (21) adjacentes au bord équatorial (3) et faisant saillie à l'intérieur de la cupule (1) pour mordre directement le noyau de glissement (26), **caractérisé en ce que** les griffes (21) sont réparties en plusieurs groupes espacés les uns des autres.

2. Cotyle selon la revendication 1, **caractérisé en ce que** les groupes de griffes (21) sont régulièrement espacés les uns des autres.

3. Cotyle selon l'une des revendications précédentes, **caractérisé en ce que** la cupule (1) comporte quatre groupes de griffes (21).

4. Cotyle selon la revendication 1, chaque groupe comporte quatre griffes (21).

5. Cotyle selon l'une des revendications précédentes, **caractérisé en ce que** la cupule (1) comporte à l'endroit du pôle (2) un taraudage central (16) débouchant et **en ce qu'**il comporte un noyau d'essai (35) qui est temporairement substituable au noyau définitif de glissement (26) et qui présente un logement interne hémisphérique (39) et comporte une partie (36) sensiblement hémisphérique conformée pour être reçue dans le logement (6) de la cupule (1) et un rebord d'appui (37) en débordement de cette cupule, le noyau temporaire d'essai possédant des moyens de sa liaison temporaire à la cupule (1) agencés pour coopérer avec le taraudage central (16) de la cupule (1) et pour permettre un pivotement avec frottement du noyau d'essai (35) dans le logement de la cupule (1) en vue de déterminer la position angulaire adéquate du noyau définitif de glissement (26).

6. Cotyle selon la revendication 5, **caractérisé en ce que** la partie sensiblement hémisphérique (36) du noyau temporaire d'essai (35) présente une dimension transversale maximum inférieure à celle du noyau définitif de glissement (26).

7. Cotyle selon l'une des revendications 5 et 6, **caractérisé en ce que** les moyens de liaison du noyau d'essai (35) à la cupule (1) comportent une vis (43) ayant une tête (45) montée librement pivotante sur le noyau d'essai (35) et une partie filetée (44) qui fait saillie à l'extérieur du noyau d'essai (35) pour être reçue dans le taraudage central (16) de la cupule (1).

8. Cotyle selon la revendication 7, **caractérisé en ce que** la tête (45) de la vis (43) de liaison temporaire est noyée dans une cavité (46) ménagée dans l'épaisseur du noyau d'essai (35) et est accessible, depuis le logement interne (39) du noyau d'essai (35), par un passage (50) débouchant dans la cavité (46).

9. Cotyle selon l'une des revendications 5 à 8, **caractérisé en ce que** le rebord (37) du noyau d'essai (35) et le rebord du noyau définitif de glissement (26) présentent tous deux un repère visuel (55, 51).

10. Cotyle selon l'une des revendications 5 à 9, **caractérisé en ce que** le taraudage central débouchant (16) est destiné à recevoir l'extrémité filetée d'un outil de montage, et est équipé d'un bouchon fileté (17) d'obturation du taraudage central (16) après montage.

11. Cotyle selon la revendication 10, **caractérisé en ce que** le bouchon fileté (17) possède une face arrière (19) qui, in situ, est située à l'intérieur de la cupule (1) et qui est pourvue d'un empreinte (20) pour coopérer avec l'extrémité correspondante d'un tournevis.

12. Cotyle selon l'une des revendications précédentes, **caractérisé en ce que** la cupule comporte extérieurement une bande annulaire cannelée (22) dont les cannelures (23, 24) sont inclinées par rapport aux méridiens de la cupule.

13. Cotyle selon la revendication 12, **caractérisé en ce que**, parmi les cannelures (23, 24) de la bande annulaire d'ancrage (23, 24), certaines sont inclinées dans un sens tandis que les autres sont inclinées dans l'autre sens.

14. Cotyle selon la revendication 13, **caractérisé en ce que** la bande annulaire d'ancrage (22) comporte au moins deux portions sur chacune desquelles les cannelures (23, 24) sont parallèles entre elles et sont inclinées en sens inverse des cannelures de l'autre portion.

15. Cotyle selon l'une des revendications 12 à 14, **caractérisé en ce que** la bande annulaire d'ancrage (22) présente des rainures annulaires (25) parallèles au bord équatorial (3).

## Claims

1. An acetabulum for a hip joint prosthesis, the acetabulum comprising:
· a cup (1) in the form of a hollow hemisphere having a pole (2) and an equatorial edge (3), and presenting an internal hemispherical housing (6); and
· a definitive slip core (26) that is received at least in part in the housing (6) of the cup (1) and that presents a hemispherical internal housing (30) for receiving a femur rod ball pressed slidably thereagainst,
the cup (1) presenting internal anchoring claws (21) adjacent to the equatorial edge (3) and projecting into the inside of the cup (1) to bite directly into the slip core (26), the acetabulum being **characterized in that** the claws (21) are distributed as a plurality of mutually spaced-apart groups.

2. An acetabulum according to claim 1, **characterized in that** the groups of claws (21) are mutually spaced apart regularly.

3. An acetabulum according to either preceding claim, **characterized in that** the cup (1) has four groups of claws (21).

4. An acetabulum according to claim 1, in which each group comprises four claws (21).

5. An acetabulum according to any preceding claim, **characterized in that** the cup (1) has a central through tapped hole (16) at the pole (2), and **in that** it includes a test core (35) which can be substituted temporarily for the definitive slip core (26) and which presents a hemispherical internal housing (39) and has a substantially hemispherical portion (36) shaped to be received in the housing (6) of the cup (1) and a bearing rim (37) projecting from said cup, the temporary test core possessing means for linking it temporarily to the cup (1) and arranged to co-operate with the central tapped hole (16) of the cup (1) and to enable the test core (35) to pivot with friction in the housing of the cup (1) in order to determine the appropriate angular position for the definitive slip core (26).

6. An acetabulum according to claim 5, **characterized in that** the substantially hemispherical portion (36) of the temporary test core (35) presents a maximum transverse dimension smaller than that of the definitive slip core (26).

7. An acetabulum according to claim 5 or claim 6, **characterized in that** the means for connecting the test core (35) to the cup (1) comprise a screw (43) having a head (45) mounted to pivot freely on the test core (35) and a threaded portion (44) projecting out from the test core (35) so as to be received in the central tapped hole (16) of the cup (1).

8. An acetabulum according to claim 7, **characterized in that** the head (45) of the temporary connection screw (43) is embedded in a cavity (46) formed in the thickness of the test core (35) and is accessible from the internal housing (39) of the test core (35) via a passage (50) opening out into the cavity (46).

9. An acetabulum according to any one of claims 5 to 8, **characterized in that** both the rim (37) of the test core (35) and the rim of the definitive slip core (26) present respective visual markers (55, 51).

10. An acetabulum according to any one of claims 5 to 9, **characterized in that** the through central tapped hole (16) is designed to receive the threaded end of a mounting tool, and is fitted with a threaded plug (17) for closing the central tapped hole (16) after mounting.

11. An acetabulum according to claim 10, **characterized in that** the threaded plug (17) possesses a rear face (19) which, in situ, is situated inside the cup (1) and is provided with a socket (20) suitable for co-operating with the corresponding end of a screwdriver.

12. An acetabulum according to any preceding claim, **characterized in that** the cup includes externally a fluted annular band (22) in which the fluting (23, 24) is inclined relative to the meridians of the cup.

13. An acetabulum according to claim 12, **characterized in that** some of the fluting (23, 24) of the anchoring annular band (23, 24) is inclined in one direction and some of it is inclined in the other direction.

14. An acetabulum according to claim 13, **characterized in that** the anchoring annular band (22) has at least two portions in each of which the fluting (23, 24) is parallel and inclined in the opposite direction to the fluting of the other portion.

15. An acetabulum according to any one of claims 12 to 14, **characterized in that** the anchoring annular band (22) presents annular grooves (25) parallel to the equatorial edge (3).

## Patentansprüche

1. Gelenkpfanne für eine Hüftgelenkprothese, umfassend:
- eine hohle, halbkugelförmige Cupula (1) mit einem Pol (2) und einem äuquatorialen Rand (3), die im Innern eine hemisphärische Aussparung (6) aufweist, und
- einen endgültigen Gleitkern (26), der zumindest teilweise in der Aussparung (6) der Cupula (1) aufgenommen ist und eine innere hemisphärische Aussparung (30) hat, um einen Gelenkkopf an dem femoralen Schaft mit Gleitkontakt aufzunehmen,
wobei die Cupula (1) innere Verankerungskrallen (21) hat, die an den äquatorialen Rand (3) angrenzen und ins Innere der Cupula (1) weisen, um direkt in den Gleitkern (26) einzugreifen, **dadurch gekennzeichnet, daß** die Krallen (21) in mehrere Gruppen eingeteilt sind, die jeweils voneinander beabstandet sind.

2. Gelenkpfanne nach Anspruch 1, **dadurch gekennzeichnet, daß** die Gruppen der Krallen (21) zueinander regelmäßigen beabstandet sind.

3. Gelenkpfanne nach einem der vorhergehenden Ansprüche, **dadurch gekennzeichnet, daß** die Cupula (1) vier Gruppen von Krallen (21) umfaßt.

4. Gelenkpfanne nach Anspruch 1, wobei jede Gruppe vier Krallen (21) umfaßt.

5. Gelenkpfanne nach einem der vorhergehenden Ansprüche, **dadurch gekennzeichnet, daß** die Cupula (1) im Bereich des Pols (2) ein zentrales nach innen geöffnetes Innengewinde (16) hat, und daß sie einen Probekern (35) umfaßt, der den endgültigen Gleitkern (26) vorübergehend ersetzen kann und der eine innere hemisphärische Aussparung (39) hat und einen im wesentlichen hemisphärischen Abschnitt (36) aufweist, der dazu geeignet ist, in der Aussparung (6) der Cupula (1) aufgenommen zu werden, und der einen überstehenden Anlagerand (37) hat, der über die Cupula hinaussteht, wobei der vorübergehende Probekern Mittel zu seiner vorübergehenden Verbindung mit der Cupula (1) hat, die derart angeordnet sind, daß sie mit dem zentralen nach innen geöffneten Innengewinde (16) der Cupula (1) zusammenwirken und eine reibschlüssige Drehbewegung des Probekerns (35) in der Aussparung der Cupula (1) ermöglichen, um die passende Winkelposition des endgültigen Gleitkerns (26) zu bestimmen.

6. Gelenkpfanne nach Anspruch 5, **dadurch gekennzeichnet, daß** der im wesentlichen hemisphärische Abschnitt (36) des vorübergehenden Probekerns (35) einen maximalen Querschnitt aufweist, der kleiner ist als der Querschnitt des endgültigen Gleitkerns (26).

7. Gelenkpfanne nach einem der Ansprüche 5 oder 6, **dadurch gekennzeichnet, daß** die Mittel zum Verbinden des Probekerns (35) mit der Cupula (1) eine Schraube (43) mit einem Kopf (45), der frei drehbar an dem Probekern (35) angeordnet ist, und mit einem Gewindeabschnitt (44) umfassen, der von der Außenseite des Probekerns (35) absteht, um in dem zentralen nach innen geöffneten Innengewinde (16) der Cupula (1) aufgenommen zu werden.

8. Gelenkpfanne nach Anspruch 7, **dadurch gekennzeichnet, daß** der Kopf (45) der Schraube (43) zur vorübergehenden Verbindung in einem Hohlraum (46) versenkt ist, der in der Dicke des Probekerns (35) ausgebildet und von der inneren Aussparung (39) des Probekerns (35) ausgehend über einen Zugang (50) zugänglich ist, der in den Hohlraum (46) mündet.

9. Gelenkpfanne nach einem der Ansprüche 5 bis 8, **dadurch gekennzeichnet, daß** der überstehende Rand (37) des Probekerns (35) und der überstehende Rand des endgültigen Gleitkerns (26) jeweils eine visuelle Markierung (55, 51) haben.

10. Gelenkpfanne nach einem der Ansprüche 5 bis 9, **dadurch gekennzeichnet, daß** das nach innen geöffnete zentrale Innengewinde (16) dazu vorgesehen ist, das mit einem Gewinde versehene Ende eines Montagewerkzeugs aufzunehmen, und nach der Montage mit einem Gewindestopfen (17) zum Verschließen des zentralen Innengewindes (16) versehen wird.

11. Gelenkpfanne nach Anspruch 10, **dadurch gekennzeichnet, daß** der Gewindestopfen (17) eine rückseitige Fläche (19) hat, die im angebrachten Zustand im Innern der Cupula (1) liegt und mit einer Einsenkung (20) versehen ist, die mit dem entsprechenden Ende eines Schraubendrehers zusammenwirken kann.

12. Gelenkpfanne nach einem der vorhergehenden Ansprüche, **dadurch gekennzeichnet, daß** die Cupula an ihrer Außenseite ein ringförmiges geriffeltes Band (22) hat, deren Riffelungen (23, 24) relativ zu den Meridianen der Cupula geneigt sind.

13. Gelenkpfanne nach Anspruch 12, **dadurch gekennzeichnet, daß** manche der Riffelungen (23, 24) des ringförmigen Verankerungsbandes in eine Richtung geneigt sind, während die anderen in eine andere Richtung geneigt sind.

14. Gelenkpfanne nach Anspruch 13, **dadurch gekennzeichnet, daß** das ringförmige Verankerungsband (22) zumindest zwei Abschnitt umfaßt, auf denen jeweils zueinander parallele Riffelungen (23, 24) angeordnet sind, die in die entgegengesetzte Richtung wie die Riffelungen des anderen Abschnitts geneigt sind.

15. Gelenkpfanne nach einem der Ansprüche 12 bis 14, **dadurch gekennzeichnet, daß** das ringförmige Verankerungsband (22) ringförmige Rillen (25) hat, die parallel zum äquatorialen Rand (3) verlaufen.
